# EUROPEAN PATENT SPECIFICATION

(11) **EP 4 011 312 B1**
(45) Date of publication and mention of the grant of the patent: **23.08.2023**
(21) Application number: 20306531.3
(22) Date of filing: 10.12.2020
(51) Int. Cl.: A61B 34/10, A61B 34/20, A61B 34/32, A61B 17/16, A61B 90/50

(54) **ROBOTIC SURGICAL SYSTEM**
ROBOTISCHES CHIRURGISCHES SYSTEM
SYSTÈME CHIRURGICAL ROBOTIQUE

(43) Date of publication of application: 15.06.2022
(73) Proprietor: DePuy Ireland Unlimited Company, County Cork (IE)
(72) Inventor: GIRARDEAU-MONTAUT, Daniel, 38610 GIERES (FR); DEMANGET, Nicolas, 38610 GIERES (FR)
(74) Representative: Regimbeau

(56) References cited:
- US-A1- 2012 143 084
- US-A1- 2014 039 517
- US-A1- 2016 135 816
- US-A1- 2017 143 432

## Description

### TECHNICAL FIELD

The present disclosure relates to a robotic surgical system configured to burr a planned volume of an anatomical structure.

### TECHNICAL BACKGROUND

Surgical interventions, for example in orthopedics, may involve removing a planned volume of an anatomical structure using a surgical burr.

The surgical burr may be held by a robotic surgical system configured to move the burr tip according to a path computed to achieve the removal of the planned volume.

Document WO 2014/198784 teaches a robotic surgical system suitable for such use.

In some circumstances, the anatomical structure to be burred is located in the vicinity of at least one another anatomical structure.

For safety reasons, it is desirable to avoid contacting the other anatomical structure with the burr, in order not to damage the other anatomical structure.

Document US 2014/0039517 teaches a robotic surgical system comprising a manipulator configured to be operated by a user.

### SUMMARY OF THE DISCLOSURE

It is thus desirable to design a robotic surgical system allowing burring an anatomical structure without damaging an adjacent anatomical structure.

Some embodiments relate to a robotic surgical system for treating an anatomical structure, comprising:
- a base configured to be handheld by a user,
- an end effector holding a surgical tool,
- an actuation unit coupled to the end effector, configured to move the end effector relative to the base,
- a tracking unit configured to determine in real time a position of the surgical tool with respect to a coordinate system of the anatomical structure,
- a control unit coupled to the tracking unit and to the actuation unit, the control unit being configured to:
   - compute a cutting path of the tool to remove a planned volume of the anatomical structure, and
   - control the actuation unit to move the surgical tool according to the computed cutting path to burr the planned volume,
wherein the control unit is configured to determine an external surface of the anatomical structure to be treated based on knowledge of said anatomical structure and to compute the tool path such that the surgical tool remains under said external surface during treatment of the planned volume so as to avoid any contact of the surgical tool with another anatomical structure.

In the present text, the term "under" defines a relative position of a volume with respect to the surface of the anatomical structure considered as an external envelope of the anatomical structure: the volume is under the surface when it is inside the envelope, whatever the orientation of the anatomical structure. To the contrary, an object is considered to be above the surface when it is outside the envelope, whatever the orientation of the anatomical structure.

In practice, it may not be possible to ensure that the tool will always remain under the external surface of the anatomical structure during the treatment of the planned volume, for example if a small volume has to be removed under the external surface; in such case, the control unit still computes the cutting path so that the tool remains as much as possible under the external surface but may issue a signal to warn the user that he may have to move at least one of the anatomical structures to avoid contacting the other anatomical structure when implementing the computed path.

In case of detection of a potential undesired contact during cutting, the control unit may stop the surgical tool, and optionally withdraw the tool in a computed direction depending on the position of at least one known anatomical structure.

By "partial mechanical link" is meant a mechanical link between at least two parts, wherein a relative movement of said at least two parts in at least one degree of freedom is possible. This term excludes a "complete" mechanical link, i.e. a link wherein no relative movement between the parts is allowed (an example of such complete mechanical link would be attaching the base rigidly to the part to be treated (e.g. a bone) by at least one screw).

As described in further detail below, said partial mechanical link provided between the base and the part of the patient's body may be direct, meaning that the support unit is in contact with the part to be treated itself, or indirect, meaning that the support unit is in contact with a part of the patient's body adjacent to the part to be treated. Said adjacent part may consist of a bone belonging to the same joint as the part to be treated, or of soft tissues that surround the part to be treated. An indirect partial mechanical link may also be obtained when the support unit is held by a user's hand and that said hand leans onto the part to be treated or the soft tissues and skin surrounding the part to be treated. Depending on the part with which the support unit makes contact and on the design of the support unit itself, said partial mechanical link may be rigid or damped.

In some embodiments, the control unit is configured to determine said external surface of the anatomical structure from palpation of the anatomical structure and/or preoperative or intraoperative imaging of said anatomical structure.

In some embodiments, the tracking unit is further configured to determine in real time a position of the surgical tool with respect to a coordinate system of the other anatomical structure.

In some embodiments, the control unit is configured to detect a potential contact between the surgical tool and the other anatomical structure and to stop the surgical tool if such a potential contact is detected.

The control unit may be configured to, if a potential contact is detected, withdraw the surgical tool in a computed direction depending on the position of at least one known anatomical structure.

In some embodiments, the control unit is configured to compute the path depending on a position of the base relative to the anatomical structure to be treated.

In some embodiments, the control unit is configured to stop the surgical tool if a current position of the surgical tool is outside the computed path.

In some embodiments, the control unit is configured to compute the path such that the planned volume is removed layer by layer.

The control unit may be configured to compute a thickness of each layer depending on a shape of the surgical tool.

In particular, the control unit may be configured to compute a first thickness for at least one first layer located at an initial surface of the anatomical structure to be treated and a second thickness for a second layer located at a final surface, the second thickness being smaller than the first thickness.

In some embodiments, the actuation unit comprises from three to five degrees of freedom in translation and/or rotation.

In some embodiments, the surgical tool is a spherical burr, a barrel burr, an oval burr, a conical burr, a straight side cutting burr, or a tapered cross-cut fissure burr.

In some embodiments, the system further comprises at least one of:
- a support unit configured to create a partial mechanical link between the base or the end effector and the anatomical structure; and
- a passive lockable arm holding the base.

In some embodiments, the control unit is configured to optimize the path such that the tool removes material from the anatomical structure depending on at least one of the following target criteria: operative time, roughness of a final surface of the treated anatomical structure, heating of the anatomical structure, wear of the surgical tool.

Some embodiments relate to a method for computing a path of a surgical tool for treating an anatomical structure, wherein the surgical tool is operated by a robotic surgical system comprising a base configured to be handheld by a user, an end effector holding the surgical tool, and an actuation unit coupled to the end effector, configured to move the end effector relative to the base, the method comprising:
- determining an external surface of the anatomical structure to be treated; and
- computing the tool path such that the surgical tool remains under said external surface so as to avoid any contact of the surgical tool with another anatomical structure.

Some embodiments relate to a robotic surgical system comprising:
- a base configured to be handheld by a user,
- an end effector holding a surgical tool,
- an actuation unit coupled to the end effector, configured to move the end effector relative to the base,
- a tracking unit configured to determine in real time a position of the surgical tool with respect to a coordinate system of the anatomical structure,
- a control unit coupled to the tracking unit and to the actuation unit, the control unit being configured to:
   - compute a path of the tool to remove a plurality of successive layers of the planned volume, the path being optimized such that the tool removes material of each layer depending on at least one of the following target criteria: operative time, roughness of a final surface of the treated anatomical structure, heating of the anatomical structure, wear of the surgical tool,
   - control the actuation unit to move the surgical tool according to the computed path to burr a planned volume of the anatomical structure, and
   - compute a first thickness for at least one first layer located at an initial surface of the anatomical structure to be treated and a second thickness for a second layer located at a final surface, the second thickness being smaller than the first thickness.

Some embodiments relate to a robotic surgical system comprising:
- a base configured to be handheld by a user,
- an end effector holding a surgical tool,
- an actuation unit coupled to the end effector, configured to move the end effector relative to the base,
- a tracking unit configured to determine in real time a position of the surgical tool with respect to a coordinate system of the anatomical structure,
- a control unit coupled to the tracking unit and to the actuation unit, the control unit being configured to:
   - compute a path of the tool to remove a plurality of successive layers of the planned volume, the path being optimized such that the tool removes material of each layer depending on at least one of the following target criteria: operative time, roughness of a final surface of the treated anatomical structure, heating of the anatomical structure, wear of the surgical tool,
   - control the actuation unit to move the surgical tool according to the computed path to burr a planned volume of the anatomical structure, and
   - detect a hard portion of the anatomical structure impeding cutting by the surgical tool, by detecting at least one of:
      - an increase of electrical current feeding motors of the actuation unit, and
      - a stationary position of the tracked surgical tool relative to the computed path.

The control unit may also be configured to stop the surgical tool upon detection of such a hard portion.

### BRIEF DESCRIPTION OF THE FIGURES

Further features and advantages of the system will be described in the following description, based on appended drawings wherein:
FIG. 1 is a general view of the system implemented during burring of a patient's bone in unicompartmental knee arthroplasty;
FIG. 2 is an enlarged view of the robotic system shown in FIG. 1;
FIGS. 3A and 3B respectively illustrate a situation where the surgical tool can remain under the surface of the anatomical structure to be treated along the whole cutting path and a situation where the surgical tool cannot fully remain under said surface along the whole cutting path;
FIG. 4A-4D show various embodiment of a surgical tool which may be used with the robotic surgical system.

### DETAILED DESCRIPTION OF EMBODIMENTS

As will be explained in further detail below, the robotic surgical system is used in a context in which a volume of the anatomical structure to be treated is planned before or during the surgical intervention.

Planning of the volume to be treated may be performed using pre-operative images (e.g. CT, MRI, Ultrasound images, 3D X-rays, PET, etc.) or intra-operative 3D data (e.g. intra-operative CT, intra-operative MRI, Ultrasound images, 2D or 3D intra-operative X-ray images, geometric data provided by localizing systems and providing 3D points, clouds of 3D points, surfaces reconstructed from clouds of 3D points, etc.), or both.

Multiple computer-assisted surgery methods may be used to register the planned volume with a coordinate system attached to the part to be treated.

Typically, intra-operative images or data are used to register pre-operative images in a unique coordinate system attached to the anatomical structure to be treated, and usually represented by a tracker (optical, magnetic, etc.).

Using any of these conventional computer-assisted surgery methods, the volume to be treated has a known geometric representation in a coordinate system attached to the anatomical structure to be treated, and whose movements are tracked in real-time by a tracking unit as it will be detailed below.

The following description is directed to unicompartmental knee arthroplasty (UKA), but is of course applicable to any other surgical procedure which requires burring an anatomical structure, and to any portion of a patient's body.

In unicompartmental knee arthroplasty, the surgeon has to remove damaged cartilage and bone from the tibia and the femur and replace the removed volumes by tibial and femoral implants. The volumes to be removed from the tibia and the femur are defined in the planning step. For example, a volume to be removed can be computed as the intersection between the anatomical structure and a prosthetic component to be implanted into said anatomical structure.

### General overview of the system

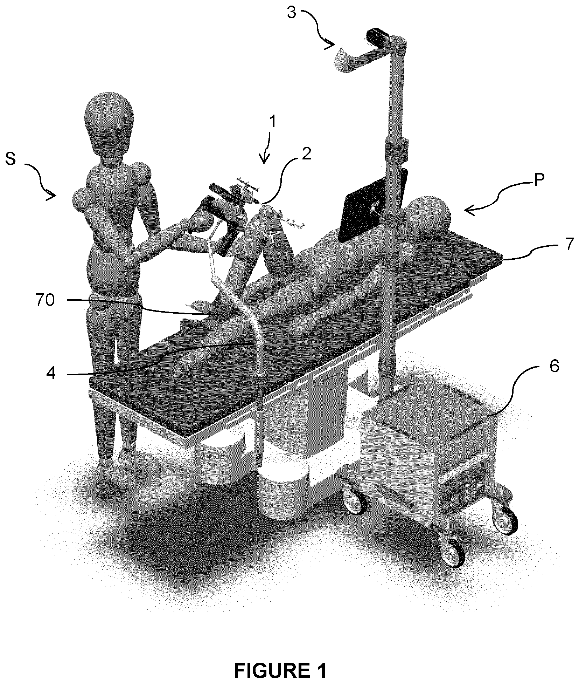

FIG. 1 shows an overview of a surgical system.

A patient P is lying on an operating table 7, with a leg to be treated maintained in flexed position by a leg holder 70.

A tool 2 which is intended to remove a 3D volume from the tibial and femoral bones is supported by a handheld device 1 that is manipulated by a user, such as a surgeon S.

The handheld device 1 is connected to a control unit 6.

Said control unit typically comprises power supply, AC/DC converters, motion controllers to power the AC/DC motors of the actuation unit, fuses, real time control system interface circuits.

The system also comprises a tracking unit 3, configured to track in real time the pose of the handheld device and the bone to be treated and to communicate tracking data to a real time control system and, if applicable, a planning system.

To that end, a coordinate system 31 is rigidly attached to the femur F, a coordinate system is rigidly attached to the tibia T, and a coordinate system 30 is rigidly attached to the tool and/or the handheld device.

In some embodiments, the tracking unit may be based on optical technology. In such case, the tracking unit may comprise a camera and the coordinate systems may each comprise a plurality of markers arranged according a respective known geometry. The camera is placed in the operating room in such a way that the coordinate systems remain within the field of view of the camera during the surgical intervention. In other embodiments, the tracking unit may be based on a non-optical technology, such as electromagnetic technology.

The tracking unit measures the relative motions between coordinate systems 30, 31, 32 at high frequencies.

The data obtained by the tracking unit is transferred to the control unit 6 via any suitable connection, with wires or wirelessly.

The real time control system is configured to carry out the proposed real time control algorithms at a reasonably high frequency.

### Handheld device

The handheld device comprises a base 1, which is intended to be hand-held by the surgeon.

To this end, a specific handle 11 may be mounted on the base, or the base may itself be designed so as to provide an ergonomic shape.

In the present text, the term "handheld" does not mean that the user is required to bear all the weight of the device. In some embodiments described below, the handheld device may be mechanically connected to the anatomical structure to be treated, and/or supported by a holding arm. However, the user still has some control on the operation of the device by holding it in his hands.

The handheld device 1 further comprises an end-effector on which the surgical tool 2 dedicated to the intended treatment can be mounted.

According to an embodiment, the tool may be factory-mounted on the end-effector; otherwise, the end-effector may comprise an attachment system (e.g. a clip-on mechanism) to secure a tool which may be a conventional tool provided separately. This embodiment has the advantage of being easily compatible with a sterile drape used to cover the actuation unit and the end-effector.

As a result, the tool 2 may or may not be part of the handheld device 1.

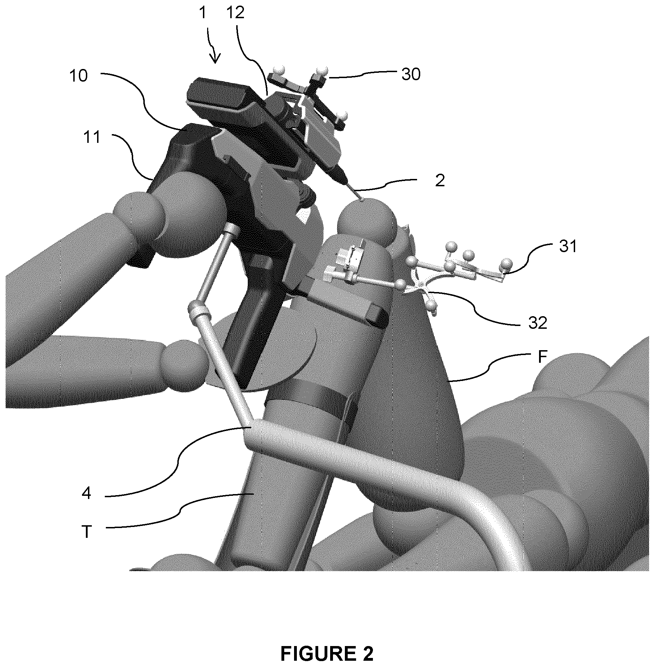

As shown in FIG. 2, the end-effector is connected to the base 10 by an actuation unit 12, in order to move the tool 2 relative to the handheld base for treating the planned volume.

The actuation unit has a given number of degrees of freedom depending on the application.

The actuation unit 12 comprises motors, gears (optional) and sensors connected together to form a kinematic structure.

As it will be explained in more detail below, the actuation unit 12 is controlled by the control unit 6.

According to one embodiment, the tool 2 may be a spherical burr or shaver and the volume to be removed may be modeled as the union of spheres having the same diameter as the burr or shaver. In such case, the actuation unit 12 may be designed so as to have three degrees of freedom (in addition to one degree of freedom to activate the rotation of the burr).

A further optimization may comprise five degrees of freedom to adjust the orientation of the cutting edges of the tool relative to the part to be treated.

In another embodiment, the tool may be a cylindrical burr or shaver and the volume to be removed may be modeled as one possible union of cylindrical segments having the same shape as the burr or shaver. In such case, the actuation unit may be designed so as to have five degrees of freedom.

The volume to be removed may also be represented by voxels, triangulated mesh data, coordinates of a plane (for sawing mode); points, directions and depth (for bore holes). And the actuation unit design will be adapted to the type of volume to be treated.

It is possible to make the actuation unit a sterile component, to be sterilized before each intervention. But, in a preferred embodiment, the actuation unit and its cables are covered by a single-use transparent plastic sterile drape. Additional components of the system can be also protected under the sterile drape. But the tool itself is sterile, like any conventional tool. Typically, the tool may be sterilized before each intervention using autoclave. Different types of mechanical adaptors between the sterile drape and the tool can be provided. Such adaptor does not require a very precise reproducible fixation if the tool contains a tracking element, which facilitates the design and the use of the global system.

### Surgical tool

In preferred embodiments, the surgical tool is a burr comprising a rotating tip.

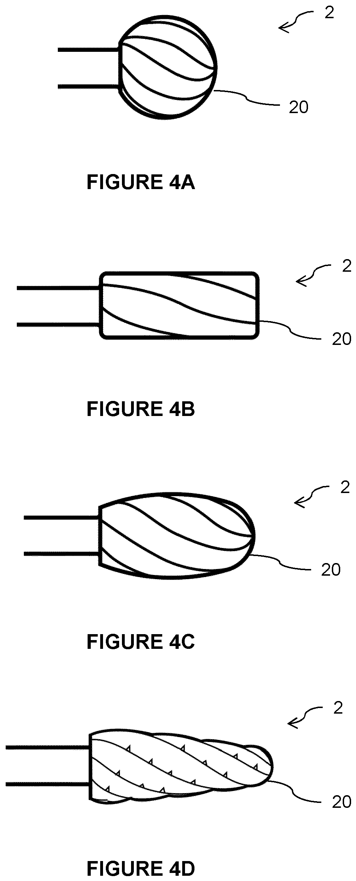

Depending on the application, the tip 20 may have various shapes, for example round (see FIG. 4A), cylindrical (see FIG. 4B), oval (see FIG. 4C), conical (see FIG. 4D). The tip may be provided with cutting blades. These examples are only provided for sake of illustration and are not limitative. Other embodiments of the burr include a straight side cutting burr and a tapered cross-cut fissure burr.

The removal of bone material is obtained by the combination of the displacement of the burr by the actuation unit and the rotation of the burr tip.

The shape of the burr tip may be chosen depending on the bone thickness to be removed during each pass of the burr.

As will be explained below, the control unit is configured to generate an optimal path of the burr tip to remove the planned volume.

### Support unit

The handheld device may further comprise a support unit 5 which may be connected either to the base or to the end-effector.

Said support unit comprises at least one element intended to make contact with the part to be treated or an area of the patient's body adjacent to the part to be treated so as to provide a partial mechanical link between the base or end-effector and the part to be treated. The user may be trained to always apply pressure to the support unit to ensure sufficient contact with the part to be treated.

The support unit is usually a sterile component. The connection between the support function and the base or end-effector may be established on the sterile drape if the actuation unit with its base and end-effector are covered with a sterile drape. Otherwise, said connection may be established directly if the base or end-effector is sterile.

The support unit acts as a stabilizer.

Said support unit may be rigid, damped (e.g. spring-loaded) and/or provide adjustable damping properties.

### Holding arm

According to one embodiment, handling of the handheld device 1 may be assisted by a holding arm 4 that supports the base of the handheld device and that is connected to a mechanical support.

The holding arm may be articulated with several degrees of freedom. The holding arm may comprise a switch used to brake or freeze the position of the arm (pneumatic arms, hydraulic arms, mechanical arms, arms with brakes, etc.). In addition, the holding arm may include a mechanism to compensate for the weight of the handheld device that it carries (by using passive or active counterweights for example).

In this case the holding arm may be considered to be a possible embodiment of the support unit previously described.

The holding arm is articulated so as to allow the user to position the handheld device 1 in a desired position.

The holding arm allows compensation for the weight of the handheld device 1 and minimizes user tiredness, especially if the surgical procedure takes a long time.

Preferably, said mechanical support is in contact with the patient's body.

The mechanical support also contributes to create a partial mechanical link between the part to be treated and the handheld device.

The mechanical support may be the operating table 7 or a support element which is commonly used to position and hold the patient (e.g. leg holder, pelvis holder, operating table post, etc.).

Additional local support may be achieved by integrating a support element on the end-effector 2 or the base 1 as described above to have a smaller force chain to compensate, with fewer vibrations. This embodiment is particularly useful if the mechanical support of the holding arm is simply a mobile cart or is ceiling-mounted.

### Generation of the cutting path

Based on the volume to be removed, the previously removed volume (if any) and the current position and orientation of the base of the robotic system with respect to the anatomical structure to be treated, the real time control unit computes an optimal tool path.

In particular, the tool path may be the path of the tool tip, without taking into account the position of the tool body.

In preferred embodiments, the tool path may be based on a layer-by-layer cutting of the volume to be treated. Otherwise said, the tool path is computed to optimally cut a plurality of successive parallel layers forming the volume to be treated.

In other embodiments, the tool path may not be constrained to cut the planned volume layer by layer but may be based on other cutting strategies.

There exist various algorithms in the robotics and machine literature for optimal milling path generation based on geometric information like binarization of the volume to be removed, or iso-parametric path generation algorithms from numerical control machining.

The computed path is optimized in that it comprises a set of positions and orientations of the tool that are computed based on current relative positions and orientations of the base of the robotic system and of the part of the anatomical structure that remains to be treated, so as to minimize at least one of the following:
- operative time;
- number of necessary repositioning actions of the base to achieve the treatment;
- heating of the anatomical structure caused by the tool;
- roughness of the surface of the anatomical structure to be achieved at the end of the treatment;
- wear of the surgical tool;
(this list is not limitative).

In some embodiments, the thickness of each layer may depend on the treatment progress. In particular, the thickness of the layers may be maximized at the beginning of the treatment, in order to minimize the operative time by removing a larger quantity of material at each pass of the tool, and be reduced at the end of the treatment, in order to reduce the roughness of the as-treated surface of the anatomical structure.

In general, the surgeon determines the surgical approach, which is the general direction through which the surgical tool accesses the anatomical structure to be treated. The approach may be selected depending on the location of the volume to be removed and of adjacent anatomical structures or tissues.

In some embodiments, it may be convenient to split the volume to be removed in two or more sub-volumes, and to determine an optimal path of the tool for treating each sub-volume. For example, if three cuts have to be made in a femur, an optimal path may be determined independently for each cut.

The selection of the surgical approach determines a position and orientation of the base of the handheld device allowing achieving the cutting path by the surgical tool. Said approach may also induce a spatial orientation of the layers to be cut when a layer-by-layer cutting of the volume to be treated is to be implemented.

In some embodiments, the control unit may determine a working space of the tool depending on the position and orientation of the base of the robotic device. Said working space corresponds to a volume accessible by the tool tip for bone removal.

Based on relative positions and orientations of the anatomical structure to be treated and of the base of the robotic device, the control unit may be configured to determine whether the working space allows removing the whole planned volume.

In a preferred embodiment, the computed path may take into account an external surface of the anatomical structure to be treated, which may be imposed as a spatial limit for the path.

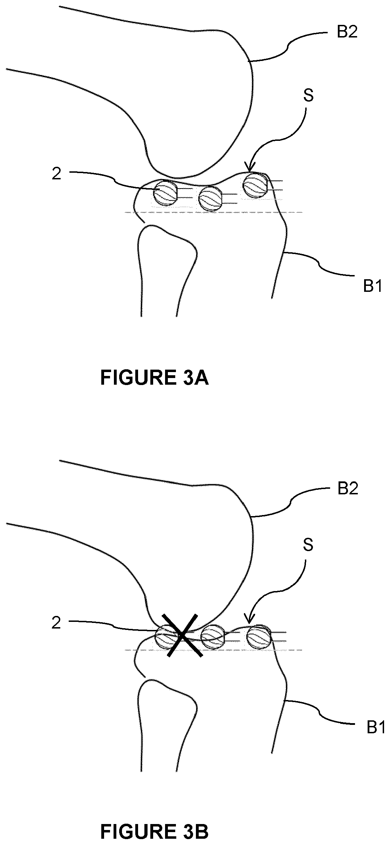

For example, as shown in FIG. 3A, the position of the external surface S of the bone B1 to be treated (for example, a tibia) may be determined by palpation using a tracked pointer.

In other embodiments, the external surface of the anatomical structure may be determined from at least one preoperative image of the anatomical structure, or at least one intraoperative image of the anatomical structure. Various intraoperative imaging methods may be used, for example echography, optical coherence tomography (OCT), fluoroscopy, etc, allowing a segmentation of the anatomical structure. Said preoperative or intraoperative image data may be registered with the anatomical structure, for example by palpating some points on the anatomical structure and associating them with corresponding points of the segmented model.

Based on such knowledge of the external surface of the anatomical structure, the control unit may thus compute the path so that the surgical tool remains below the surface S, in order to prevent any contact with an adjacent bone B2 (for example, a femur), located above the surface S. The dotted line represents the final surface to be achieved.

This control of the cutting path is different from a haptic guiding. In haptic devices, virtual boundaries are defined that cannot be crossed by the surgical tool, the movement of the tool being free within said boundaries. In the present robotic system, the movement of the tool is constrained to follow the computed cutting path.

In the above embodiment, it is not required to know the position of the adjacent anatomical structure to compute the cutting path.

In other embodiments, the control unit may take into account the position of another anatomical structure, adjacent to the anatomical structure to be treated, to compute the cutting path in such a way as to avoid any contact of the surgical tool with said other anatomical structure.

In some situations (see FIG. 3B), if the bone thickness between the initial surface S and the final surface is too thin, the selected tool may be too large to allow implementing the path without contacting the anatomical structure B2. In such case, the control unit may generate a signal (e.g. a message) to the user to select a smaller tool. Alternatively, for example if it is not desired or possible to select another tool, the control unit may compute the path so that the tool remains as much as possible under the external surface, and generate a signal to the user to warn him of said risk of interference, so that the user may move at least one of the anatomical structures to increase reachability of the anatomical structure to be treated during the treatment. For example, in the case of knee arthroplasty, the user may mobilize the knee in extension or in flexion to move the tibia away from the femur.

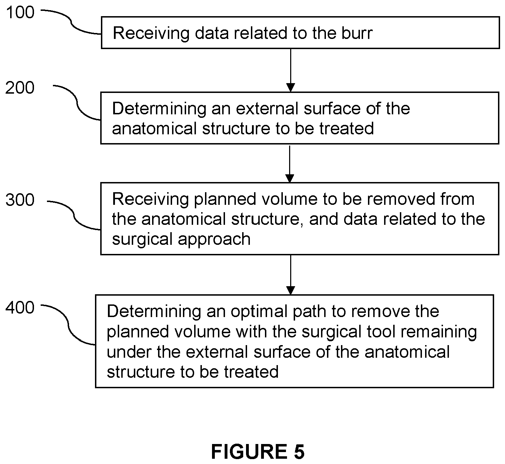

One embodiment of a method implemented by the control unit to compute the optimal path is illustrated in FIG. 5.

In step 100, the control unit receives data about the burr to be used for the surgical treatment. Said data may include the shape (e.g. round, cylindrical, oval, conical, etc.), size, and number of blades of the burr. This data may be input by a user through a user interface.

In step 200, the control unit receives data regarding the surface of the anatomical structure. As explained above, said data may be obtained by palpation or by preoperative or intraoperative imaging. Said data may be uploaded by the user through the user interface.

In step 300, the control unit receives planning data including the volume to be removed from the anatomical structure. Said planning data may also include a priori information about the approach selected by the surgeon and constraints related to the environment of the anatomical structure. This planning data may be input or uploaded by the user through the user interface; otherwise, the planning may be generated automatically thanks to previously acquired anatomy data.

It is to be noted that steps 100, 200 and 300 may be implemented simultaneously or in a different order from the one represented in FIG. 5.

In step 400, the control unit computes the cutting path so as to ensure that the surgical tool remains under the external surface of the anatomical structure during the treatment.

If such a constraint is not possible to meet for the whole treatment, for example due to the fact that the surgical tool is too large to always remain under the external surface of the anatomical structure to be treated, the control unit may generate an alert to the user and request that at least one parameter (e.g. shape or size of the tool, approach...) be changed. Then, the control unit computes a new cutting path based on the new data. Alternatively, the control unit may compute the cutting path so that the tool remains as much as possible under the external surface, e.g. minimizing the portions of the cutting path where at least a part of the tool is above the external surface. The control unit may thus generate an alert to the user to pay attention to the risk of interference with the other anatomical structure during the treatment. In such case, the user may have to move at least one of the anatomical structures to increase reachability of the anatomical structure to be treated if he sees or feels that the tool is close to the other anatomical structure.

### Implementation of the generated cutting path

Once the cutting path has been computed, a signal may be sent to the user to inform him that he may trigger operation of the robotic device and of the surgical tool. For safety reasons, the surgical tool may not be operated until the cutting path has been generated.

During burring, the anatomical structure and the robotic device are tracked relative to each other in real time.

The control unit is configured to determine in real time whether the tool is located at the expected position along the computed path.

In case of a deviation of the tool from the computed path by a determined threshold, the control unit may stop the surgical tool (e.g. turning off the burr) and command the actuation unit to bring the surgical tool to the desired position on the cutting path. Such a deviation may typically be caused by the latency of the components of the robotic system. The surgical tool may thus remain stopped until the tool tip is again close enough to the cutting path.

### Detection of a risk of conflict with the adjacent anatomical structure

During burring, the adjacent anatomical structure may also be tracked in real time relative to the anatomical structure being treated and the robotic device.

The control unit may thus be configured to detect a risk of interaction of the tool with said adjacent anatomical structure in a next step of the path. In order to anticipate such an interaction, the control unit may stop the surgical tool (e.g. turn off the burr). Once the surgical tool has been stopped, the control unit may additionally control the actuation unit to withdraw the tool in a computed direction depending on the position of the adjacent anatomical structure in order not to contact it during the withdrawal.

Said position of the adjacent anatomical structure may be known by tracking coordinate systems attached to the anatomical structure to be treated and to the other anatomical structure.

Alternatively, the position of the adjacent anatomical structure may be assumed, based on a priori knowledge of the situation. For example, in knee arthroplasty, the user generally pushes the patella on the same side when burring the tibia, and it is thus possible to infer a limiting plane not to be crossed by the tool during the withdrawal to avoid contacting the patella.

Then, the control unit may generate a new cutting path of the surgical tool to remove the remaining bony material.

### Detection of hard or elastic regions of the anatomical structure

In some situations, the surgical tool may encounter hard regions (e.g. sclerotic bone) and/or elastic regions (e.g. meniscus) of the anatomical structure to be treated.

The tool may not be able to cut such hard or elastic regions. As a result, the tool may thus remain stationary relative to the anatomical structure and thereby generate a large amount of heat and wear quickly.

In order to detect such situations and prevent excessive heating of the anatomical structure, the control unit may implement various methods.

One of these methods is based on the detection of an immobilization of the robotic system via localization data. To that end, as mentioned above, a coordinate system is attached to the surgical tool or to the actuation unit and tracked by the tracking unit. Based on tracking data regarding the anatomical structure and the surgical tool, the control unit may detect that the surgical tool is no longer advancing and/or is not at the expected position along the generated cutting path.

Another method is based on the level of electrical currents in the motors of the actuation unit. Indeed, when the tool encounters an obstacle which cannot be cut, the electrical current that feeds the motors of the actuation unit increases in order to continue displacing the tool along the computed cutting path. Threshold values of said electrical currents may be determined to detect the presence of such an obstacle. The control unit may monitor the value of electrical currents feeding the motors of the actuation unit and detect a hard or elastic region in the anatomical structure if at least one of said electrical currents exceeds a respective threshold.

Another method is based on the level of pressure exerted by the tool onto the anatomical structure, measured by a pressure sensor, which may typically be arranged on the tool. Threshold values of said pressure may be determined to detect the presence of such an obstacle. The control unit may monitor the value of pressure measured by the pressure sensor and detect a hard or elastic region in the anatomical structure if said pressure exceeds a predetermined threshold.

Of course, the above methods may be combined.

As a result of said detection, the control unit may stop the tool (e.g. turn off the burr) in order to prevent further heating of the anatomical structure.

In some embodiments, the control unit may also control the actuation unit to retract the surgical tool to avoid contact between the surgical tool and the anatomical structure. Said retraction may be of a few millimeters in order to reduce heating of the anatomical structure by the tool.

The control unit may also be configured to issue a warning signal (e.g. a visual and/or audible signal) to the user, requesting the user to remove the hard or elastic region in order to allow the robotic system continuing the treatment. The removal of the hard or elastic obstacle may be carried out by any suitable means, e.g. a cutting tool manually operated by the user.

### REFERENCES

WO 2014/198784

## Claims

1. Robotic surgical system for treating an anatomical structure (B1), comprising:
- a base (10) configured to be handheld by a user,
- an end effector (13) holding a surgical tool (2),
- an actuation unit (12) coupled to the end effector, configured to move the end effector relative to the base,
- a tracking unit (3) configured to determine in real time a position of the surgical tool with respect to a coordinate system of the anatomical structure,
- a control unit (6) coupled to the tracking unit and to the actuation unit, the control unit being configured to:
• compute a path of the tool (2) to remove a planned volume of the anatomical structure, and
• control the actuation unit to move the surgical tool according to the computed path to burr the planned volume,
**characterised in that**
the control unit is configured to determine an external surface (S) of the anatomical structure (B1) to be treated and to compute the tool path such that the surgical tool remains under said external surface (S) during treatment of the planned volume so as to avoid any contact of the surgical tool with another anatomical structure (B2).

2. System according to claim 1, wherein the control unit is configured to determine said external surface (S) of the anatomical structure (B1) from palpation of the anatomical structure (B1) and/or preoperative or intraoperative imaging of said anatomical structure (B1).

3. System according to claim 1 or claim 2, wherein the tracking unit is further configured to determine in real time a position of the surgical tool with respect to a coordinate system of the other anatomical structure (B2).

4. System according to any one of claims 1 to 3, wherein the control unit is configured to detect a potential contact between the surgical tool and the other anatomical structure (B2) and to stop the surgical tool (2) if such a potential contact is detected.

5. System according to claim 5, wherein the control unit is configured to, if a potential contact is detected, withdraw the surgical tool (2) in a computed direction depending on the position of at least one known anatomical structure.

6. System according to any one of claims 1 to 6, wherein the control unit is configured to compute the path depending on a position of the base (10) relative to the anatomical structure (B1) to be treated.

7. System according to any one of claims 1 to 7, wherein the control unit is configured to stop the surgical tool (2) if a current position of the surgical tool is outside the computed path.

8. System according to any one of claims 1 to 7, wherein the control unit is configured to compute the path such that the planned volume is removed layer by layer.

9. System according claim 8, wherein the control unit is configured to compute a thickness of each layer depending on a shape of the surgical tool.

10. System according to claim 9, wherein the control unit is configured to compute a first thickness for at least one first layer located at an initial surface of the anatomical structure (B1) to be treated and a second thickness for a second layer located at a final surface, the second thickness being smaller than the first thickness.

11. System according to any one of claims 1 to 10, wherein the actuation unit (12) comprises from three to five degrees of freedom in translation and/or rotation.

12. System according to any one of claims 1 to 11, wherein the surgical tool (2) is a spherical burr, a barrel burr, an oval burr, a conical burr, a straight side cutting burr, or a tapered cross-cut fissure burr.

13. System according to any one of claims 1 to 12, further comprising at least one of:
- a support unit configured to create a partial mechanical link between the base or the end effector and the anatomical structure; and
- a passive lockable arm (4) holding the base.

14. System according to any one of claims 1 to 13, wherein the control unit is configured to optimize the path such that the tool removes material from the anatomical structure depending on at least one of the following target criteria: operative time, roughness of a final surface of the treated anatomical structure, heating of the anatomical structure, wear of the surgical tool.

15. Method for computing a path of a surgical tool for treating an anatomical structure, wherein the surgical tool is operated by a robotic surgical system comprising a base (10) configured to be handheld by a user, an end effector (13) holding the surgical tool (2), and an actuation unit (12) coupled to the end effector, configured to move the end effector relative to the base, **characterised in that** the method comprises:
- determining an external surface (S) of the anatomical structure (B1) to be treated; and
- computing the tool path such that the surgical tool remains under said external surface (S) so as to avoid any contact of the surgical tool with another anatomical structure (B2).

## Patentansprüche

1. Robotergestütztes chirurgisches System zur Behandlung einer anatomischen Struktur (B1), umfassend:
- eine Basis (10), die so konfiguriert ist, dass sie von einem Benutzer in der Hand gehalten werden kann
- einen Endeffektor (13), der ein chirurgisches Werkzeug (2) hält
- eine mit dem Endeffektor gekoppelte Betätigungseinheit (12), die dazu eingerichtet ist, den Endeffektor relativ zum Sockel zu bewegen
- eine Verfolgungseinheit (3), die so eingerichtet ist, dass sie in Echtzeit eine Position des chirurgischen Werkzeugs in Bezug auf ein Koordinatensystem der anatomischen Struktur bestimmt
- eine Steuereinheit (6), die mit der Verfolgungseinheit und der Betätigungseinheit gekoppelt ist, wobei die Steuereinheit so konfiguriert ist, dass sie:
• einen Pfad des Werkzeugs (2) berechnet, um ein geplantes Volumen der anatomischen Struktur zu entfernen, und
• die Betätigungseinheit steuert, um das chirurgische Werkzeug entsprechend dem berechneten Weg zum Fräsen des geplanten Volumens zu bewegen,
**dadurch gekennzeichnet, dass**
die Steuereinheit so konfiguriert ist, dass sie eine Außenfläche (S) der zu behandelnden anatomischen Struktur (B1) bestimmt und den Werkzeugweg so berechnet, dass das chirurgische Werkzeug während der Behandlung des geplanten Volumens unter dieser Außenfläche (S) bleibt, um jeglichen Kontakt des chirurgischen Werkzeugs mit einer anderen anatomischen Struktur (B2) zu vermeiden.

2. System nach Anspruch 1, wobei die Steuereinheit so eingerichtet ist, dass sie die Außenfläche (S) der anatomischen Struktur (B1) durch Palpation der anatomischen Struktur (B1) und/oder präoperative oder intraoperative Bildgebung der anatomischen Struktur (B1) bestimmt.

3. System nach Anspruch 1 oder Anspruch 2, wobei die Verfolgungseinheit ferner dazu eingerichtet ist, in Echtzeit eine Position des chirurgischen Werkzeugs in Bezug auf ein Koordinatensystem der anderen anatomischen Struktur (B2) zu bestimmen.

4. System nach einem der Ansprüche 1 bis 3, wobei die Steuereinheit dazu eingerichtet ist, einen potenziellen Kontakt zwischen dem chirurgischen Werkzeug und der anderen anatomischen Struktur (B2) zu erkennen und das chirurgische Werkzeug (2) anzuhalten, wenn ein solcher potenzieller Kontakt erkannt wird.

5. System nach Anspruch 5, wobei die Steuereinheit so eingerichtet ist, dass sie, wenn ein potenzieller Kontakt erkannt wird, das chirurgische Werkzeug (2) in einer berechneten Richtung in Abhängigkeit von der Position mindestens einer bekannten anatomischen Struktur zurückzieht.

6. System nach einem der Ansprüche 1 bis 6, wobei die Steuereinheit dazu eingerichtet ist, den Weg abhängig von einer Position der Basis (10) relativ zur zu behandelnden anatomischen Struktur (B1) zu berechnen.

7. System nach einem der Ansprüche 1 bis 7, wobei die Steuereinheit dazu eingerichtet ist, das chirurgische Werkzeug (2) anzuhalten, wenn eine aktuelle Position des chirurgischen Werkzeugs außerhalb des berechneten Pfades liegt.

8. System nach einem der Ansprüche 1 bis 7, wobei die Steuereinheit dazu eingerichtet ist, den Weg so zu berechnen, dass das geplante Volumen Schicht für Schicht entfernt wird.

9. System nach Anspruch 8, wobei die Steuereinheit so eingerichtet ist, dass sie eine Dicke jeder Schicht in Abhängigkeit von einer Form des chirurgischen Werkzeugs berechnet.

10. System nach Anspruch 9, wobei die Steuereinheit so eingerichtet ist, dass sie eine erste Dicke für wenigstens eine erste Schicht berechnet, die sich an einer Anfangsfläche der zu behandelnden anatomischen Struktur (B1) befindet, und eine zweite Dicke für eine zweite Schicht, die sich an einer Endfläche befindet, wobei die zweite Schichtdicke kleiner als die erste Schichtdicke ist.

11. System nach einem der Ansprüche 1 bis 10, wobei die Betätigungseinheit (12) drei bis fünf Freiheitsgrade in Verschiebung und/oder Drehung umfasst.

12. System nach einem der Ansprüche 1 bis 11, wobei das chirurgische Werkzeug (2) ein Kugelfräser, ein Zylinderfräser, ein ovaler Fräser, ein konischer Fräser, ein gerader Schneidfräser oder ein konischer Kreuzschnitt-Rissfräser ist.

13. System nach einem der Ansprüche 1 bis 12, ferner umfassend wenigstens eines der Folgenden:
- eine Stützeinheit, die so ausgelegt ist, dass sie eine teilweise mechanische Verbindung zwischen der Basis oder dem Endeffektor und der anatomischen Struktur herstellt; und
- ein passiver arretierbarer Arm (4), der die Basis hält.

14. System nach einem der Ansprüche 1 bis 13, wobei die Steuereinheit so eingerichtet ist, dass sie den Weg so optimiert, dass das Werkzeug Material von der anatomischen Struktur entfernt, abhängig von mindestens einem der folgenden Zielkriterien: Operationszeit, Rauigkeit einer Endoberfläche der behandelten anatomischen Struktur, Erwärmung der anatomischen Struktur, Verschleiß des chirurgischen Werkzeugs.

15. Verfahren zur Berechnung eines Pfads eines chirurgischen Werkzeugs zur Behandlung einer anatomischen Struktur, wobei das chirurgische Werkzeug von einem robotergestützten chirurgischen System bedient wird, das eine Basis (10) umfasst, die vom Benutzer gehalten werden kann, einen Endeffektor (13), der das chirurgische Werkzeug (2) hält, und eine mit dem Endeffektor gekoppelte Betätigungseinheit (12), die dazu eingerichtet ist, den Endeffektor relativ zur Basis zu bewegen, **dadurch gekennzeichnet, dass** das Verfahren umfasst:
- Bestimmung einer äußeren Oberfläche (S) der zu behandelnden anatomischen Struktur (B1); und
- Berechnung des Werkzeugwegs so, dass das chirurgische Werkzeug unter der Außenfläche (S) verbleibt, um jeglichen Kontakt des chirurgischen Werkzeugs mit einer anderen anatomischen Struktur (B2) zu vermeiden.

## Revendications

1. Système chirurgical robotisé pour le traitement d'une structure anatomique (B1), comprenant :
- une base (10) configurée pour être tenue à la main par un utilisateur,
- un effecteur (13) tenant un outil chirurgical (2),
- une unité d'actionnement (12) couplée à l'effecteur, configurée pour déplacer l'effecteur par rapport à la base,
- une unité de suivi (3) configurée pour déterminer en temps réel une position de l'outil chirurgical par rapport à un système de coordonnées de la structure anatomique,
- une unité de commande (6) couplée à l'unité de suivi et à l'unité d'actionnement, l'unité de commande étant configurée pour :
• calculer une trajectoire de l'outil (2) pour enlever un volume planifié de la structure anatomique, et
• commander l'unité d'actionnement pour déplacer l'outil chirurgical selon la trajectoire calculée afin de fraiser le volume prévu,
**caractérisé par le fait que** l'unité de commande est configurée pour déterminer une surface externe (S) de la structure anatomique (B1) à traiter et pour calculer la trajectoire de l'outil de manière à ce que l'outil chirurgical reste sous ladite surface externe (S) pendant le traitement du volume prévu afin d'éviter tout contact de l'outil chirurgical avec une autre structure anatomique (B2).

2. Système selon la revendication 1, dans lequel l'unité de commande est configurée pour déterminer ladite surface externe (S) de la structure anatomique (B1) à partir de la palpation de la structure anatomique (B1) et/ou de l'imagerie préopératoire ou peropératoire de ladite structure anatomique (B1).

3. Système selon la revendication 1 ou la revendication 2, dans lequel l'unité de suivi est en outre configurée pour déterminer en temps réel une position de l'outil chirurgical par rapport à un système de coordonnées de l'autre structure anatomique (B2).

4. Système selon l'une des revendications 1 à 3, dans lequel l'unité de commande est configurée pour détecter un contact potentiel entre l'outil chirurgical et l'autre structure anatomique (B2) et pour arrêter l'outil chirurgical (2) si un tel contact potentiel est détecté.

5. Système selon la revendication 5, dans lequel l'unité de commande est configurée pour, si un contact potentiel est détecté, retirer l'outil chirurgical (2) dans une direction calculée en fonction de la position d'au moins une structure anatomique connue.

6. Système selon l'une des revendications 1 à 6, dans lequel l'unité de commande est configurée pour calculer la trajectoire en fonction d'une position de la base (10) par rapport à la structure anatomique (B1) à traiter.

7. Système selon l'une des revendications 1 à 7, dans lequel l'unité de commande est configurée pour arrêter l'outil chirurgical (2) si une position actuelle de l'outil chirurgical est en dehors de la trajectoire calculée.

8. Système selon l'une des revendications 1 à 7, dans lequel l'unité de commande est configurée pour calculer la trajectoire de manière à ce que le volume prévu soit enlevé couche par couche.

9. Système selon la revendication 8, dans lequel l'unité de commande est configurée pour calculer l'épaisseur de chaque couche en fonction de la forme de l'outil chirurgical.

10. Système selon la revendication 9, dans lequel l'unité de commande est configurée pour calculer une première épaisseur pour au moins une première couche située à une surface initiale de la structure anatomique (B1) à traiter et une seconde épaisseur pour une seconde couche située à une surface finale, la seconde épaisseur étant inférieure à la première épaisseur.

11. Système selon l'une des revendications 1 à 10, dans lequel l'unité d'actionnement (12) comprend de trois à cinq degrés de liberté en translation et/ou en rotation.

12. Système selon l'une des revendications 1 à 11, dans lequel l'outil chirurgical (2) est une fraise sphérique, une fraise cylindrique, une fraise ovale, une fraise conique, une fraise à coupe latérale droite ou une fraise fissure conique à coupe transversale.

13. Système selon l'une des revendications 1 à 12, comprenant en outre au moins l'un des éléments suivants :
- une unité de support configurée pour créer une liaison mécanique partielle entre la base ou l'effecteur et la structure anatomique ; et
- un bras passif verrouillable (4) qui maintient la base.

14. Système selon l'une des revendications 1 à 13, dans lequel l'unité de commande est configurée pour optimiser la trajectoire de manière à ce que l'outil enlève de la matière de la structure anatomique en fonction d'au moins un des critères cibles suivants : temps de fonctionnement, rugosité d'une surface finale de la structure anatomique traitée, échauffement de la structure anatomique, usure de l'outil chirurgical.

15. Méthode de calcul de la trajectoire d'un outil chirurgical pour traiter une structure anatomique, dans laquelle l'outil chirurgical est utilisé par un système chirurgical robotisé comprenant une base (10) configurée pour être tenue à la main par un utilisateur, un effecteur (13) tenant l'outil chirurgical (2), et une unité d'actionnement (12) couplée à l'effecteur, configurée pour déplacer l'effecteur par rapport à la base, **caractérisée en ce que** la méthode comprend :
- déterminer une surface externe (S) de la structure anatomique (B1) à traiter ; et
- calculer la trajectoire de l'outil de manière à ce que l'outil chirurgical reste sous ladite surface externe (S) afin d'éviter tout contact de l'outil chirurgical avec une autre structure anatomique (B2).
